# EUROPÄISCHE PATENTANMELDUNG

(11) **EP 0 653 636 A2**
(43) Veröffentlichungstag der Anmeldung: **17.05.1995**
(21) Anmeldenummer: 94117652.1
(22) Anmeldetag: 09.11.1994
(51) Int. Cl.: G01N 33/52, G01J 1/50, G01N 33/48, G01N 31/22

(54) **Teststreifen zur Bestimmung der UV-Intensität und des Risikos eines Sonnenbrandes**

(30) Priorität: 15.11.1993 DE 4338811
(71) Anmelder: BOEHRINGER MANNHEIM GMBH, D-68298 Mannheim (DE)
(72) Erfinder: Krause, Manfred, D-68519 Viernheim (DE); Kaufmann, Norbert, Dr., D-67152 Ruppertsberg (DE); Neumann, Mathias, Dr.,, D-69469 Weinheim (DE)

(57) **Zusammenfassung**

Gegenstand der Erfindung ist die Verwendung von Teststreifen mit einer flächigen, bevorzugt länglichen Folie, die eine Matrix mit einer photoaktiven chromogenen Substanz trägt, zur Vorbestimmung der möglichen sonnenbrandfreien Aufenthaltsdauer in der Sonne,
ein Testsystem für diese Bestimmung enthaltend Teststreifen und eine Teststreifenpackung,
eine Teststreifenpackung und
ein Verfahren zur Vorbestimmung der maximal möglichen sonnenbrandfreien Aufenthaltsdauer in der Sonne mit dem Testsystem.

## Beschreibung

Die Erfindung betrifft die Verwendung von Teststreifen zur Bestimmung der UV-Intensität oder Vorbestimmung der sonnenbrandfreien Aufenthaltsdauer in der Sonne. Weiter betrifft die Erfindung ein entsprechend geeignetes Testsystem mit Teststreifen und Teststreifenpackung. Die Erfindung betrifft ebenfalls ein Verfahren zur Vorbestimmung der maximal möglichen sonnenbrandfreien Aufenthaltsdauer in der Sonne.

Intensive kurzwellige Ultraviolettstrahlung, wie sie im Sonnenlicht enthalten ist, kann nicht nur zu Lichterythemen, Sonnenbrand und Hautnekrosen führen, sondern gegebenenfalls auch zu Hautkrebs, wenn die Einwirkung der schädigenden Strahlung lang genug andauert. Man glaubt eine erhöhte Hautkrebsinzidenz mit der Ozonverarmung der Troposphäre in Verbindung bringen zu können. Personen, die sich Sonnenlicht aussetzen müssen, wie beispielsweise Personen, die im Freien arbeiten, tragen deshalb diesbezüglich ein erhöhtes Gesundheitsrisiko. Andererseits wird Hautbräunung durch Sonnenlicht weitverbreitet als ein Merkmal von Gesundheit angesehen. Dies trifft insbesondere für hellhäutige Bewohner der Erde zu, welche durch Sonnenbaden ihre Haut zu bräunen suchen.

Seit sich ein Bewußtsein für die gesundheitsgefährdenden Wirkungen des Sonnenlichts in breiten Kreisen der Bevölkerung ausgebildet hat, wird nach Lösungen gesucht, die die aktuelle UV-Intensität mit einer Aussage korrelieren, nach welcher Sonneneinwirkungsdauer eine Person mit einem Sonnenbrand zu rechnen hat.

Es gibt hierfür relativ teuere Geräte, die beispielsweise wie eine Uhr am Handgelenk getragen werden, die die aktuelle UV-Intensität integrativ messen und bei Überschreiten der zulässigen Zeit ein Warnsignal geben. Außer dem hohen Preis besitzen solche Geräte den Nachteil, daß am Armgelenk bei aufrechter Haltung des Trägers deutlich geringere Werte durch nicht-senkrechten Strahlungseinfall gemessen werden als bei liegender Stellung des Trägers mit Ausrichtung des Gerätes zur Sonne oder auf exponierten Hautflächen wie beispielsweise Nase oder Schultern.

Photoaktive Substanzen, die je nach UV-Intensität und Expositionszeit ihre Farben reversibel oder irreversibel ändern, sind bekannt. Reversible Substanzen haben hierbei den Nachteil, daß sie nach bestimmten Expositionszeiten ihre Reaktionsfähigkeit verlieren und deshalb nicht mehr in den ursprünglichen Farbzustand zurückkehren. Der Test muß deshalb immer lichtgeschützt aufbewahrt werden. Außerdem ist die Farbbildung solcher Substanzen temperaturabhängig, was bei gleicher UV-Intensität zu sehr großen Ergebnisunterschieden führt, wenn beispielsweise Messungen mit der gleichen Substanz im Hochgebirge oder am Strand durchgeführt werden.

Substanzen, die irreversible Farbveränderungen bei UV-Einwirkung erfahren, sind von auf die Haut zu klebenden Etiketten bekannt. Diese Ausführungsformen besitzen den Nachteil, daß beim Sonnenbaden helle Flecken an den Klebestellen zurückbleiben. Vor allem weiß der Anwender bei solchen Tests nicht im voraus, wie lange er sich der Sonne aussetzen darf, sondern nur, wann er das Limit erreicht hat. Eine Vorplanung ist bei diesem Test nicht möglich. Werden die Klebeetiketten, die die phototaktive Substanz tragen, auf Körperstellen geklebt, die dem Sonnenlicht exponiert sind, wie Gesicht, Nase oder Schultern, dann sind diese Etiketten nicht oder nur sehr schwer durch den Träger selbst ablesbar, ohne sie von der Körperpartie zu entfernen. Bei jedem Entfernen des Etiketts läßt die Klebekraft jedoch nach, so daß entweder das Etikett nicht mehr wie vorgesehen angeklebt werden kann oder wenn man das Abnehmen des Etiketts deshalb vermeiden möchte, die Gefahr nicht ausschließen kann, daß man den Zeitpunkt übersieht, an dem vor einem Sonnenbrand gewarnt wird.

Aufgabe der vorliegenden Erfindung war es deshalb, Mittel zur Verfügung zu stellen, mit denen einfach und preiswert die UV-Intensität bestimmt werden kann und eine Vorbestimmung der maximal möglichen sonnenbrandfreien Aufenthaltsdauer in der Sonne durchführbar ist.

Diese Aufgabe wird durch den Gegenstand der Erfindung, wie er in den Ansprüchen gekennzeichnet ist, gelöst.

Gegenstand der Erfindung ist die Verwendung von Teststreifen mit jeweils einer flächigen, bevorzugt länglichen Folie, die eine Matrix mit einer photoaktiven chromogenen Substanz trägt zur Bestimmung der UV-Intensität von Licht, insbesondere zur Vorbestimmung der möglichen sonnenbrandfreien Aufenthaltsdauer in der Sonne.

Gegenstand der Erfindung ist auch ein Testsystem zur Bestimmung der UV-Intensität enthaltend Teststreifen, die im wesentlichen jeweils aus einer flächigen, bevorzugt länglichen Folie bestehen, die eine Matrix mit einer photoaktiven, chromogenen Substanz trägt, wobei die Teststreifen gegebenenfalls vor Licht geschützt, einzeln verpackt vorliegen können und eine Teststreifenpackung, die zur Aufnahme von Teststreifen geeignet ist, welche im wesentlichen jeweils aus einer flächigen, bevorzugt länglichen Folie bestehen, die eine Matrix mit einer photoaktiven chromogenen Substanz trägt. Die erfindungsgemäße Teststreifenpackung besteht im wesentlichen aus einem Behältnis, welches zur Aufnahme der Teststreifen geeignet ist und aus einer Vorrichtung, die das Aufhängen der Teststreifenpackung auf einen Stift ermöglicht. Die Packung ist dadurch gekennzeichnet, daß das Behältnis eine Schachtel ist, die im wesentlichen die Form eines Quaders mit den Kanten a, b und c und mit sechs begrenzenden, ebenen Flächen aufweist, wobei die Kanten a, b und c alle gleich oder verschieden lang sein können oder auch zwei gleich und eine hiervon verschieden lang sein kann. Dementsprechend können auch die sechs begrenzenden, ebenen Flächen alle gleich wie in einem Würfel oder paarweise verschieden sein, wie in einem gestauchten Würfel (zwei der Kanten a, b und c sind gleich) oder in einem Quader, der nur von rechteckigen, nicht quadratischen Flächen begrenzt ist (Kanten a, b und c sind alle verschieden). Eine der den Quader begrenzenden ebenen Flächen reicht über den Quader hinaus und trägt die Aufhängevorrichtung. Außerdem ist die der Aufhängevorrichtung nächstliegende reguläre Kante des Quaders durch zwei zueinander und zu der regulären Kante des Quaders im wesentlichen parallele Kanten vollständig oder teilweise ersetzt. Aufhängevorrichtung und eine oder beide parallelen Kanten stehen in einem solchen geometrischen Verhältnis zueinander, daß eine von einer oder beiden parallelen Kanten durch die Aufhängevorrichtung zielende Gerade einen bestimmten Winkel mit der über die Quadergrenzen hinausreichenden ebenen Fläche bildet.

Weiter ist Gegenstand der Erfindung eine Teststreifenpackung, wie sie vorstehend als Teil des erfindungsgemäßen Testsystems beschrieben worden ist.

Schließlich ist Gegenstand der Erfindung ein Verfahren zur Vorbestimmung der maximal möglichen sonnenbrandfreien Aufenthaltsdauer in der Sonne mit einem Testsystem, wie es vorstehend beschrieben worden ist, dadurch gekennzeichnet, daß ein Teststreifen aus einer flächigen, bevorzugt länglichen Folie, die eine Matrix mit einer photoaktiven chromogenen Substanz trägt, für eine bestimmte Zeit dem Sonnenlicht ausgesetzt und anschließend mit einer Vergleichsskala mit verschiedenen Farbfeldern verglichen wird, worauf von dem Farbfeld der Vergleichsskala das der Farbe der Matrix mit der photoaktiven chromogenen Substanz am nächsten kommt die maximal mögliche sonnenbrandfreie Aufenthaltsdauer in der Sonne abgelesen werden kann.

Im folgenden wird die Erfindung anhand von einen möglichen Ausführungsweg darstellenden Zeichnungen näher erläutert. Es zeigen:
Figur 1a) einen Teststreifen zur Bestimmung der UV-Intensität und zur Vorbestimmung der möglichen sonnenbrandfreien Aufenthaltsdauer in der Sonne,
Figur 1b) einen in Verpackungsmaterial einzeln verpackten Teststreifen,
Figur 2) eine Teststreifenpackung,
Figur 3) einen Querschnitt durch die Teststreifenpackung gemäß Figur 2) entlang der Linie A-A mit einer Geraden durch die Aufhängevorrichtung über Kanten e und d,
Figur 4) eine weitere Ausführungsform einer Teststreifenpackung und
Figur 5) eine Teststreifenpackung mit zur Durchführung einer erfindungsgemäßen Bestimmung aufgelegtem Teststreifen.

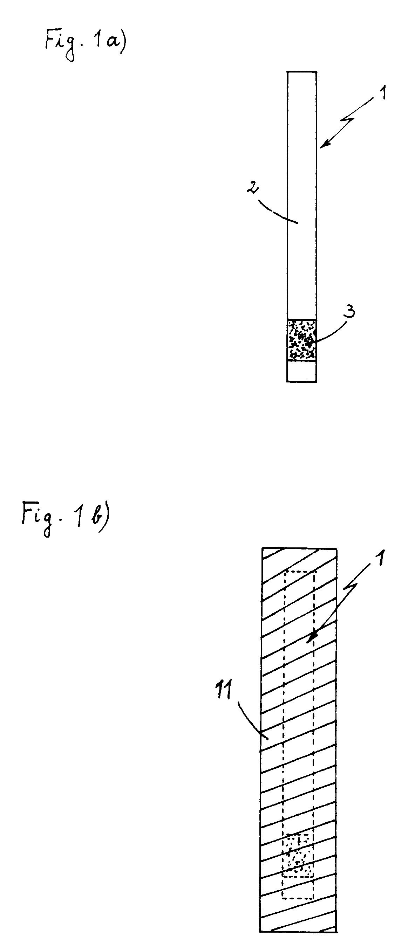

Erfindungsgemäß sollen zur Bestimmung der aktuellen UV-Intensität in Licht und Vorbestimmung der möglichen sonnenbrandfreien Aufenthaltsdauer in der Sonne Teststreifen eingesetzt werden, wie beispielsweise der in Figur 1 a) dargestellte. Solche Teststreifen bestehen im wesentlichen jeweils aus einer flächigen, bevorzugt länglichen, insbesondere rechteckigen, Folie (2), die eine Matrix (3) mit einer photoaktiven chromogenen Substanz trägt. Von der Struktur her sind solche Teststreifen aus der analytischen Chemie schon längere Zeit, beispielsweise als Blut- oder Harnteststreifen, bekannt. Während solche Teststreifen ganz unterschiedliche Dimensionen besitzen können, haben sich für die erfindungsgemäß eingesetzten Teststreifen solche als bevorzugt erwiesen, die zwischen ca. 2 und 10 cm lang und zwischen ca. 3 und 15 mm, vorzugsweise zwischen ca. 4 und 8 mm breit sind. Als Folie wird jedes Material bezeichnet, das relativ dünn ist und dennoch eine solche Steifigkeit besitzt, daß es zur bestimmungsgemäßen Handhabung geeignet ist. So können solche Folien aus Holz, Glas, Metall oder Kunststoff bestehen. Besonders bevorzugt sind Kunststoffolien, wie beispielsweise Polystyrol, Polyester oder Polyethylen, die etwa zwischen 0,2 und 0,5 mm dick sind.

Die Folie (2) trägt eine Matrix aus einem saugfähigen oder quellfähigen Material. Poröse oder nichtporöse Filme oder Membranen, Vliese, Gewirke oder Gewebe kommen in Frage. Besonders geeignet hat sich im erfindungsgemäßen Umfeld eine Matrix aus einem Polyester-Vlies erwiesen. Das Matrixmaterial als Teil des Teststreifens kann zwischen ca. 0,1 und 0,8 mm dick sein und eine rechteckige oder quadratische Fläche zwischen ca. 9 und 250 mm² aufweisen. Besonders bevorzugt sind Matrixmaterialien, die zwischen ca. 0,3 und 0,6 mm dick und zwischen ca. 16 und 36 mm² groß sind. Während es grundsätzlich möglich ist, diese Matrix (3) an jeder beliebigen Stelle einer Folie (2) aufzubringen hat es sich für den erfindungsgemäßen Zweck als besonders geeignet erwiesen, die Matrix (3) an oder nahe einem Ende einer länglichen Folie (2) zu befestigen. Die Befestigung kann durch Verklebung der Matrix mit der Folie erfolgen oder auch durch ein Einspannen der Matrix zwischen der Folie und einem darüber gespannten Material. In letzterem Fall ist natürlich darauf zu achten, daß das darüber gespannte Material für UV-Licht durchlässig ist. Besonders bevorzugt ist jedoch das Verkleben der Matrix (3) mit der Folie (2). Dies kann direkt, beispielsweise mittels eines Schmelzklebers zwischen Matrix und Folie, beispielsweise durch thermisches Aufschmelzen einer klebenden Schicht einer zu diesem Zweck beschichteten Folie, geschehen, oder auch durch ein Doppelklebeband zwischen Matrix und Folie. Letzteres ist eine ganz bevorzugte Ausführungsform.

Die Matrix (3) des erfindungsgemäß einsetzbaren Teststreifen (1) trägt eine photoaktive Substanz, das heißt eine Substanz, die sich bei der Einwirkung von Sonnenlicht verändert. Vorzugsweise werden für die vorliegende Erfindung solche Substanzen eingesetzt, die bei der Einwirkung von Sonnenlicht ihre Farbe verändern. Solche Substanzen werden als chromogen bezeichnet, wobei unter einer Farbveränderung sowohl der Wechsel von einer Farbe zu einer anderen verstanden wird als auch das Bilden einer Farbe von einer zuvor farblosen Substanz und auch das Farbloswerden einer zuvor farbigen Substanz. Erfindungsgemäß haben sich solche Substanzen als besonders gut geeignet erwiesen, die gegenüber Feuchte und Temperatureinflüssen stabil sind und die je nach Sonnenlichtintensität innerhalb bestimmter Zeiträume visuell gut erkennbare Farbabstuffingen liefern. Natürlich sind solche Substanzen ganz besonders bevorzugt, die selektiv die sogenannte UV-B Strahlung (Wellenlängen zwischen 280 und 320 nm) und UV-A Strahlung (Wellenlängen zwischen 320 und 400 nm), insbesondere kurzwellige UV-A-Strahlung, absorbieren. Hervorragend geeignet ist in diesem Sinne 2,18-Phosphormolybdänsäure als photoaktive chromogene Substanz, die ihre Farbe je nach Sonnenlichtintensität und Belichtungsdauer von hellgelb über hellblau nach dunkelblau hin ändert. Teststreifen mit dieser Substanz sind aus EP-A-0 431 456 bekannt zur kolorimetrischen Bestimmung von Glucose in Körperflüssigkeit. Die Substanz wird dort als 18-Molybdodiphosporsäure bezeichnet.

Erfindungsgemäß kann die photoaktive chromogene Substanz auf die Matrix (3) beschichtet sein. Vorzugsweise wird die Matrix (3) jedoch mit einer Lösung oder Suspension der Substanz in einem geeigneten Lösungsmittel oder Dispergiermittel imprägniert.

Da die Teststreifen zur Vorbestimmung der möglichen sonnenbrandfreien Aufenthaltsdauer in der Sonne eine photoaktive Substanz beinhalten, ist es empfehlenswert, die Teststreifen lichtgeschützt aufzubewahren. Vorteilhafterweise werden die Teststreifen (1) gemäß Figur 1b) so verpackt in den Verkehr gebracht, daß kein Licht auf die photoaktive Substanz trifft. Außerdem sollen die Teststreifen während ihrer Aufbewahrung vor Feuchtigkeit geschützt sein. Vorzugsweise wird deshalb jeder Teststreifen einzeln verpackt in Verpackungsmaterial (11) in den Handel gebracht. Als Verpackungsmaterial (11) haben sich feuchtigkeits- und lichtdichte Alufolien als vorteilhaft erwiesen, mit denen die Teststreifen (1) einzeln verpackt werden können. In Figur 1 b) ist ein verpackter Teststreifen (1) dargestellt. Dieser befindet sich zwischen zwei lichtundurchlässigen Folien, die um den Teststreifen herum miteinander verbunden sind und so den Teststreifen (1) allseitig umschließen und gegenüber Umwelteinflüssen, insbesondere Licht und Feuchtigkeit isolieren.

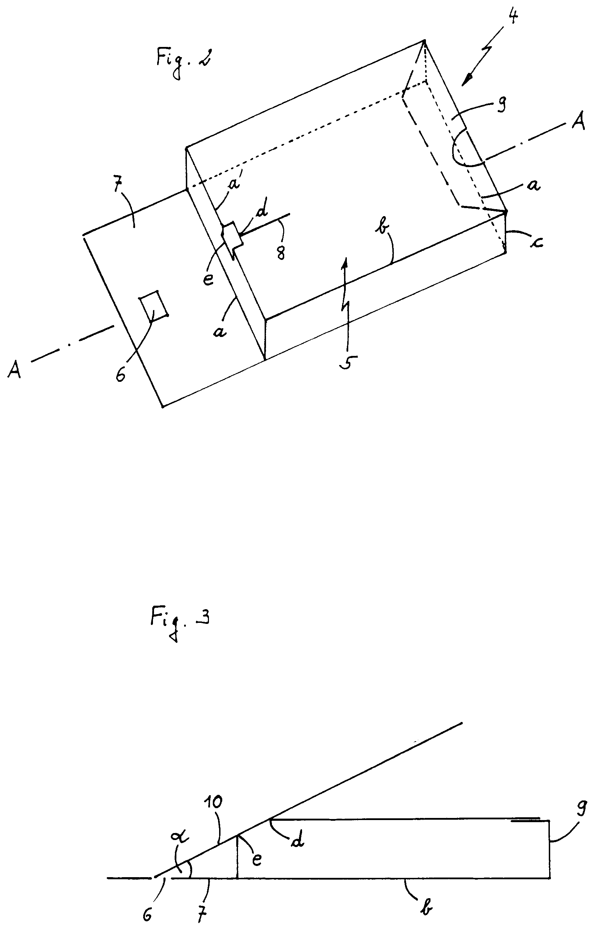

Ein erfindungsgemäßes Testsystem zur Vorbestimmung der sonnenbrandfreien Aufenthaltsdauer in der Sonne enthält vorstehend beschriebene Teststreifen, vorzugsweise in Verpackungsmaterial (11) einzeln verpackt, und eine Teststreifenpackung, welche zur Aufnahme der Teststreifen geeignet ist, wobei die Teststreifenpackung ein Behältnis zur Aufnahme der Teststreifen beinhaltet und eine Vorrichtung, die das Aufhängen der Teststreifenpackung auf einen Stift ermöglicht. Eine erfindungsgemäße Teststreifenpackung (4) ist in Figur 2 dargestellt. Sie umfaßt ein Behältnis (5) in Form einer Schachtel, die im wesentlichen die Form eines Quaders mit den Kanten a), b) und c) aufweist. Ein Quader ist normalerweise dadurch gekennzeichnet, daß er durch 6 ebene Flächen begrenzt ist, wobei die Kanten a), b) und c) alle gleich oder verschieden lang sein können oder auch zwei gleich und eine hiervon verschieden lang sein können. Im Falle, daß alle drei Kanten gleich lang sind, handelt es sich um einen Kubus, bei dem alle Begrenzungsflächen quadratisch sind. Wenn alle drei Kanten verschieden lang sind, handelt es sich bei den 6 Begrenzungsflächen um jeweils drei Paare von rechteckigen Flächen, wobei jedes Paar aus zwei gleichen rechteckigen Flächen besteht, die drei Paare im Vergleich miteinander jedoch unterschiedliche rechteckige Flächen aufweisen.

In der erfindungsgemäßen Teststreifenpackung (4) reicht eine der den Quader begrenzenden ebenen Flächen über den Quader hinaus (7). Diese Fläche trägt eine Aufhängevorrichtung (6), die das Aufhängen der Teststreifenpackung (4) auf einen Stift ermöglicht. Als besonders vorteilhaft hat sich als Aufhängevorrichtung (6) ein Loch erwiesen. Ganz besonders bevorzugt ist ein Loch als Aufhängevorrichtung (6), das so gestaltet ist, daß ein Teststreifen (1) wie er vorstehend beschrieben worden ist, in dieses Loch paßt. Die Dimensionierung dieses Loches sollte vorteilhafterweise so sein, daß ein Hindurchführen des Streifens durch das Loch möglich ist und der Teststreifen hierbei eine gewisse Führung erfährt. Das heißt, der Teststreifen kann in seiner Längsrichtung durch das Loch so hindurchgezogen werden kann, daß beide Längsseiten des Teststreifens Lochwände streifen.

In dem Behältnis (5) der erfindungsgemäßen Teststreifenpackung (4) ist die der Aufhängevorrichtung (6) nächstliegende reguläre Kante des Quaders (a') durch zwei zueinander und zu der regulären Kante des Quaders im wesentlichen parallele Kanten (d, e) vollständig oder teilweise ersetzt. Beispielsweise kann man sich vorstellen, daß ein teilweiser Ersatz der regulären Kante des Quaders (a') dadurch erreicht wird, daß ein Stück dieser Kante herausgeschnitten worden ist. Ein solcher teilweiser Ersatz der regulären Kante (a') ist besonders bevorzugt.

In der erfindungsgemäßen Teststreifenpackung (4) stehen die Aufhängevorrichtung (6) und eine oder beide der parallelen Kanten (d, e) in einem solchen geometrischen Verhältnis zueinander, daß eine von einer oder beiden parallelen Kanten durch die Aufhängevorrichtung zielende Gerade (10) einen bestimmten Winkel (α) mit der über die Quadergrenzen hinausreichenden, ebenen Fläche (7) bildet. Dies ist in Figur 3 schematisch dargestellt. Die Gerade (10) berührt dort beide parallelen Kanten (d, e) und die Aufhängevorrichtung (6). Erfindungsgemäß ist es jedoch auch möglich, daß die Gerade (10) nur auf einer der Kanten (d,e) aufliegt. Dies ist dann vorstellbar, wenn die Kante (d) nicht hoch genug ist, um die auf Kante (e) aufliegende Gerade zu berühren oder wenn Kante (e) nicht hoch genug ist, um die auf Kante (d) aufliegende Gerade zu berühren. In jedem Fall bildet die Gerade (10) einen bestimmten Winkel (α ) mit der über die Quadergrenze hinausreichenden, ebenen Fläche (7). Dieser Winkel (α) beträgt zwischen ca. 10 und 40 Grad, vorzugsweise zwischen ca. 20 und 30 Grad.

Besonders bevorzugt ist eine erfindungsgemäße Packung (4) bei der das Behältnis (5) eine quaderförmige Schachtel ist, bei der mindestens eine der Grenzflächen als Klappe (9) ausgebildet ist. Diese Klappe (9) kann die Form einer Lasche haben, die zum Entnehmen von Teststreifen aus dem Behältnis (5) aufgeklappt werden kann und anschließend durch Zurückschieben der Lasche wieder verschlossen werden kann. Vorzugsweise ist die der Aufhängevorrichtung am entferntesten angeordneten Grenzfläche als eine solche Klappe (9) ausgebildet.

Als Material für die Teststreifenpackung (4) kann grundsätzlich jedes Material verwendet werden, das eine gewisse Eigensteifigkeit aufweist, so daß die Form der Teststreifenpackung nach ihrer Herstellung auch erhalten bleibt. Denkbare Materialien sind vor allem Kunststoff und Papier, wobei sich Papier, insbesondere Karton als besonders bevorzugt erwiesen hat, da dieses Material sich nach entsprechendem Zuschnitt durch Faltung und Kleben leicht in die entsprechende Form bringen läßt.

Papier, insbesondere Karton hat sich auch deshalb als bevorzugt erwiesen, weil dieses Material gut bedruckt werden kann. So kann beispielsweise leicht auf die der über den Quader hinausreichenden Fläche gegenüberliegende Fläche ein Strich (8) aufgebracht werden, der zur Aufhängevorrichtung (6) weist. Dieser Strich sollte vorzugsweise so angeordnet sein, daß er der Projektion der von der Aufhängevorrichtung (6) über die Kanten (d, e) hinausragenden Geraden (10) auf die durch die Kanten a- und b gebildeten Fläche, das heißt die der über den Quader hinausreichenden Fläche gegenüberliegende Fläche, entspricht.

Da die Teststreifenpackung (4) zur Aufnahme von Teststreifen zur Vorbestimmung der möglichen sonnenbrandfreien Aufenthaltsdauer in der Sonne geeignet sein soll, ist es klar, daß die Maße der Teststreifenpackung (4) den Teststreifendimensionen entsprechend angepaßt sein muß. Als vorteilhaft für das Behältnis (5) haben sich Kantenlängen für a zwischen ca. 70 und 90 mm, für b zwischen ca. 150 und 170 mm und für c zwischen ca. 15 und 25 mm erwiesen. Vorteilhafterweise ist der kürzeste Abstand der Aufhängevorrichtung (6) von der nächsten Begrenzungsfläche des quaderförmigen Behältnisses (5) etwa 40 mm. Der für die Teststreifenpackung (4) besonders bevorzugt eingesetzte Karton hat eine Stärke von etwa 0,35 mm. Ist die der Aufhängevorrichtung (6) nächstliegende reguläre Kante des Quaders (a') durch zwei zueinander und zu der regulären Kante des Quaders im wesentlichen parallele Kanten teilweise ersetzt, so beträgt die jeweilige Länge der beiden parallelen Kanten (d, e) zwischen ca. 3 und 15 mm, vorzugsweise zwischen ca. 4 und 8 mm. Sie sind der Breite der Teststreifen angepaßt.

Die erfindungsgemäß einsetzbaren Teststreifen erlauben es, die aktuelle UV-Intensität von Licht zu bestimmen, da sich bei verschiedenen UV-Intensitäten die Farbe der Matrix des Teststreifens innerhalb einer gegebenen Zeitdauer unterschiedlich verändert. Mittels einer Farbvergleichsskala, die bei bekannten UV-Intensitäten erstellt wurde, können so auch unbekannte UV-Intensitäten ermittelt werden.

Das erfindungsgemäße Verfahren zur Vorbestimmung der maximal möglichen sonnenbrandfreien Aufenthaltsdauer in der Sonne wird so durchgeführt, daß ein Teststreifen der vorstehend beschriebenen Art, aus einer flächigen, bevorzugt länglichen Folie, die eine Matrix mit einer photoaktiven chromogenen Substanz trägt für eine bestimmte Zeit dem Sonnenlicht ausgesetzt wird. Dadurch ergibt sich einen Farbveränderung der photoaktiven chromogenen Substanz in der Matrix. Die resultierende Farbveränderung wird mit einer Farbvergleichsskala verglichen. Europide Menschen können bezüglich ihres Hautbräunungsverhaltens und ihrer Empfindlichkeit auf Sonnenbrand in 4 Hauttypen unterteilt werden:

| ***Hauttyp*** | ***Hautreaktion und ethnische Zuordnung*** |
|---|---|
| I | Immer schnell Sonnenbrand, kaum oder keine Bräunung auch nach wiederholter Bestrahlung (keltischer Typ) |
| II | Fast immer Sonnenbrand, mäßige Bräunung nach wiederholten Bestrahlungen (hellhäutiger europäischer Typ) |
| III | Mäßig oft Sonnenbrand, fortschreitende Bräunung nach wiederholten Bestrahlungen (dunkelhäutiger europäischer Typ) |
| IV | Selten Sonnenbrand, schnell einsetzende und deutliche Bräunung (mittelmeerischer Typ) |

Diese Typenunterteilung findet weltweit Anwendung. Eine Anleitung für die Ermittlung des Hauttyps einer bestimmten Person ist in dem erfindungsgemäßen Testsystem ebenfalls vorteilhafterweise enthalten.

Wegen der 4 möglichen unterschiedlichen Hauttypen sollte das erfindungsgemäße Testsystem Farbvergleichsskalen für die verschiedenen Hauttypen enthalten. Für einen Hauttyp sind dort verschiedene Farbabstufungen abgebildet, die mögliche Farben der die photoaktive chromogene Substanz enthaltenden Matrix nach einer bestimmten Expositionsdauer darstellen. Jede Farbe entspricht einer bestimmten UV-Intensität, aus der sich herleiten läßt, wie lange eine Person eines bestimmten Hauttyps sich in der Sonne aufhalten kann, ohne einen Sonnenbrand zu bekommen. Insofern ist jedem Farbfeld der Farbvergleichsskala eine Zeitangabe zugeordnet, die die mögliche sonnenbrandfreie Aufenthaltsdauer in der Sonne angibt. Das praktische Vorgehen beim Verfahren zur Vorbestimmung der maximal möglichen sonnenbrandfreien Aufenthaltsdauer in der Sonne ist deshalb so, daß bei Kenntnis des Hauttyps einer Person die entsprechende Farbvergleichsskala mit der Farbe der Matrix des Teststreifens verglichen wird, der eine bestimmte Zeit dem Sonnenlicht ausgesetzt worden ist. Die Farbe des Feldes der Farbvergleichsskala, die der Farbe der Matrix mit der photoaktiven chromogenen Substanz am nächsten kommt, gibt dann die Zeit an, während der eine sonnenbrandfreie Aufenthaltsdauer in der Sonne möglich ist.

Insbesondere Teststreifen mit 2,18-Phosphormolybdänsäure berücksichtigen die für die Hautalterung verantwortlich UV-A-Strahlung. Auf der Farbvergleichsskala wird diese Strahlung bezüglich der Zeitangabe berücksichtigt. Ohne diese Berücksichtigung könnten die empfohlenen Zeitangaben um etwa 40 bis 60% größer sein.

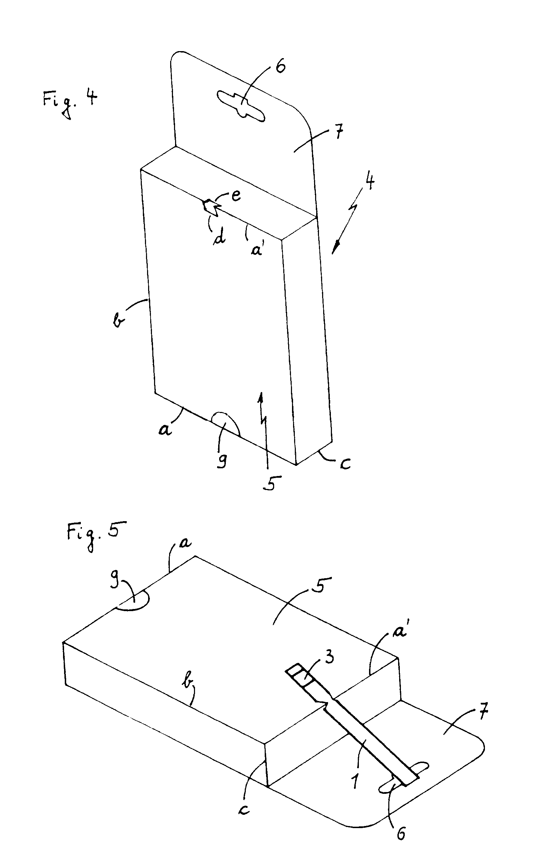

Besonders vorteilhaft wird das erfindungsgemäße Verfahren so ausgeführt, daß der Teststreifen so zur Sonne ausgerichtet wird, daß der Lichteinfall möglichst senkrecht zu der Matrix mit der photoaktiven chromogenen Substanz erfolgt. Eine solche Ausrichtung kann besonders einfach mit Hilfe der erfindungsgemäßen Teststreifenpackung erfolgen, die auf einen möglichst waagrechten Untergrund, wie beispielsweise Erdboden oder Tischfläche, gelegt wird. Der Teststreifen wird so auf eine oder beide parallelen Kanten, die die reguläre Quaderkante (a') des Behältnisses der Teststreifenpackung ersetzt, gelegt, daß er in die Aufhängevorrichtung der Teststreifenpackung reicht. Der Teststreifen wird so in einer Neigung gehalten, daß möglichst viel Licht auf die Matrix mit der photoaktiven chromogenen Substanz erfolgt. Eine entsprechende Ausführung ist in Figur 5) dargestellt. Ganz besonders vorteilhaft ist es zur Ausrichtung der Teststreifenpackung mit dem eingelegten Teststreifen zur Sonne, den auf der über den Quader hinausreichenden Fläche gegenüberliegenden Fläche befindlichen Strich auszunutzen. Der eingelegte Teststreifen kann nämlich wie der Zeiger einer Sonnenuhr wirken. Die Packung sollte so gedreht werden, daß der Schatten des Teststreifens mit dem Strich auf der Schachtelfläche zur Deckung kommt, dann ist der Streifen optimal ausgerichtet.

Der Vorteil des erfindungsgemäßen Testsystems besteht darin, preiswert, unter vielen typischen Urlaubsbedingungen eine individuelle Vorhersage zur ungefährlichen Aufenthaltsdauer in der Sonne zu machen. Das Testsystem ist klein und handlich, so daß es praktisch immer mitgeführt werden kann. Durch die Möglichkeit der Ausrichtung des Teststreifens zur Sonne mit Hilfe der Teststreifenpackung ist es möglich, in Gegenden zwischen ca. 60° nördlicher und 60° südlicher Breite leicht und reproduzierbar die Zeitdauer vorherzubestimmen, die man sich in der Sonne aufhalten kann, ohne einen Sonnenbrand befürchten zu müssen. Unabhängig von der geographischen Breite nimmt die kurzwellige UV-Strahlung auch sehr stark mit der Höhe über dem Meeresspiegel zu. Diese Zunahme kann bis zu 20% pro 1000 m betragen. Außerdem kommt es bei UV-Strahlung und deren Wirkung auf Menschen auch auf die Topographie und Bodenbeschaffenheit des Meßortes an. Insofern ist das erfindungsgemäße Testsystem auch vorteilhaft im Berg- und Wintersport einzusetzen.

Wenn auf die Vorteile der Teststreifenpackung verzichtet werden soll, ist natürlich erfindungsgemäß auch ein Testsystem möglich, das Teststreifen zur Bestimmung der UV-Intensität und zur Vorbestimmung der möglichen sonnenbrandfreien Aufenthaltsdauer in der Sonne, im wesentlichen bestehend aus einer flächigen, bevorzugt länglichen Folie (2), die eine Matrix (3) mit einer photoaktiven, chromogenen Substanz trägt, gegebenenfalls ein Verpackungsmaterial (11) einzelverpackt und eine Farbvergleichsskala mit mehreren Farbfeldern, wobei den einzelnen Farbfeldern Zahlenwerte zugeordnet sind, die die mögliche sonnenbrandfreie Aufenthaltsdauer einer Person in der Sonne angeben, enthält. Die Teststreifen, deren mögliche Einzelverpackung und die Farbvergleichsskala sowie deren Anwendung entsprechen völlig den zuvor beschriebenen. Insofern können auch in einem solchen vereinfachten Testsystem mehrere Farbvergleichsskalen enthalten sein, die jeweils auf einzelne Hauttypen ausgerichtet sind.

## Patentansprüche

1. Verwendung von Teststreifen mit einer flächigen, bevorzugt länglichen Folie, die eine Matrix mit einer photoaktiven, chromogenen Substanz trägt zur Bestimmung der UV-Intensität oder zur Vorbestimmung der möglichen sonnenbrandfreien Aufenthaltsdauer in der Sonne.

2. Testsystem zur Vorbestimmung der sonnenbrandfreien Aufenthaltsdauer in der Sonne enthaltend
Teststreifen zur Bestimmung der UV-Intensität und zur Vorbestimmung der möglichen sonnenbrandfreien Aufenthaltsdauer in der Sonne, im wesentlichen bestehend aus einer flächigen, bevorzugt länglichen Folie (2), die eine Matrix (3) mit einer photoaktiven chromogenen Substanz trägt, gegebenenfalls in Verpackungsmaterial (11) einzelverpackt, und
eine Teststreifenpackung mit einem Behältnis (5), welches zur Aufnahme der gegebenenfalls einzelverpackten Teststreifen geeignet ist und
einer Vorrichtung (6), die das Aufhängen der Teststreifenpackung auf einen Stift ermöglicht, wobei
das Behältnis (5) eine Schachtel ist, die im wesentlichen die Form eines Quaders mit den Kanten a, b und c und mit 6 begrenzenden, ebenen Flächen aufweist, wobei die Kanten a, b und c alle gleich oder verschieden lang sein können oder auch zwei gleich und eine hiervon verschieden lang sein können und eine der den Quader begrenzenden ebenen Flächen über den Quader hinausreicht (7) und die Aufhängevorrichtung (6) trägt und außerdem die der Aufhängevorrichtung (6) nächstliegende reguläre Kante des Quaders (a') durch 2 zueinander und zu der regulären Kante des Quaders im wesentlichen parallele Kanten (d,e) vollständig oder teilweise ersetzt ist, wobei Aufhängevorrichtung (6) und eine oder beide der parallelen Kanten (d,e) in einem solchen geometrischen Verhältnis zueinander stehen, daß eine von einer oder beiden parallelen Kanten durch die Aufhängevorrichtung zielende Gerade (10) einen bestimmten Winkel (α) mit der über die Quadergrenzen hinausreichenden, ebenen Fläche (7) bildet.

3. Testsystem gemäß Anspruch 2, dadurch gekennzeichnet, daß es zusätzlich eine Farbvergleichsskala enthält.

4. Testsystem gemäß einem der Ansprüche 2 oder 3, dadurch gekennzeichnet, daß es eine Beilage zur Anleitung für die Ermittlung eines Hauttyps enthält.

5. Testsystem zur Vorbestimmung der sonnenbrandfreien Aufenthaltsdauer in der Sonne enthaltend
Teststreifen zur Bestimmung der UV-Intensität und zur Vorbestimmung der möglichen sonnenbrandfreien Aufenthaltsdauer in der Sonne, im wesentlichen bestehend aus einer flächigen, bevorzugt länglichen Folie (2), die eine Matrix (3) mit einer photoaktiven chromogenen Substanz trägt, gegebenenfalls in Verpackungsmaterial (11) einzeln verpackt, und
mindestens eine Farbvergleichsskala mit mehreren Farbfeldern, wobei den einzelnen Farbfeldern Zahlenwerte zugeordnet sind, die die mögliche sonnenbrandfreie Aufenthaltsdauer einer Person in der Sonne angeben.

6. Testsystem gemäß Anspruch 5, dadurch gekennzeichnet, daß es mehrere Farbvergleichsskalen enthält, die jeweils auf einzelne Hauttypen ausgerichtet sind.

7. Teststreifenpackung (4), geeignet zur Aufnahme von gegebenenfalls einzelverpackten Teststreifen (1) zur Bestimmung der UV-Intensität und zur Vorbestimmung der möglichen sonnenbrandfreien Aufenthaltsdauer in der Sonne, im wesentlichen bestehend aus einer flächigen, bevorzugt länglichen Folie (2), die eine Matrix (3) mit einer photoaktiven chromogenen Substanz trägt mit einem Behältnis (5), welches zur Aufnahme der gegebenenfalls einzelverpackten Teststreifen geeignet ist und
einer Vorrichtung (6), die das Aufhängen der Teststreifenpackung auf einen Stift ermöglicht, dadurch gekennzeichnet, daß
das Behältnis (5) eine Schachtel ist, die im wesentlichen die Form eines Quaders mit den Kanten a, b und c und mit 6 begrenzenden, ebenen Flächen aufweist, wobei die Kanten a, b und c alle gleich oder verschieden lang sein können oder auch zwei gleich und eine hiervon verschieden lang sein können und eine der den Quader begrenzenden ebenen Fläche über den Quader hinausreicht (7) und die Aufhängevorrichtung (6) trägt und außerdem die der Aufhängevorrichtung (6) nächstliegende reguläre Kante des Quaders (a') durch 2 zueinander und zu der regulären Kante des Quaders im wesentlichen parallele Kanten (d,e) vollständig oder teilweise ersetzt ist, wobei Aufhängevorrichtung (6) und eine oder beide der parallelen Kanten (d,e) in einem solchen geometrischen Verhältnis zueinander stehen, daß eine von einer oder beiden parallelen Kanten durch die Aufhängevorrichtung zielende Gerade (10) einen bestimmten Winkel (α) mit der über die Quadergrenzen hinausreichenden, ebenen Fläche (7) bildet.

8. Teststreifenpackung gemäß Anspruch 7, dadurch gekennzeichnet, daß die Aufhängevorrichtung (6) ein Loch ist.

9. Teststreifenpackung gemäß Anspruch 7, dadurch gekennzeichnet, daß der Winkel (α) zwischen ca. 10 ° und 40 ° liegt.

10. Teststreifenpackung gemäß Anspruch 7, dadurch gekennzeichnet, daß die Schachtel eine Klappe (9) besitzt.

11. Teststreifenpackung gemäß Anspruch 7, dadurch gekennzeichnet, daß die der über den Quader hinausreichenden Fläche (7) gegenüberliegende Fläche einen Strich (8) aufgedruckt enthält, der zur Aufhängevorrichtung (6) weist.

12. Verfahren zur Vorbestimmung der maximal möglichen sonnenbrandfreien Aufenthaltsdauer in der Sonne mit dem Testsystem gemäß einem der Ansprüche 2 bis 6, dadurch gekennzeichnet, daß
ein Teststreifen aus einer flächigen, bevorzugt länglichen Folie, die eine Matrix mit einer photoaktiven chromogenen Substanz trägt für eine bestimmte Zeit dem Sonnenlicht ausgesetzt und anschließend mit einer Vergleichsskala mit verschiedenen Farbfeldern verglichen wird, worauf von dem Farbfeld der Vergleichsskala, das der Farbe der Matrix mit der photoaktiven chromogenen Substanz am nächsten kommt die maximal mögliche sonnenbrandfreie Aufenthaltsdauer in der Sonne abgelesen werden kann.

13. Verfahren gemäß Anspruch 12, dadurch gekennzeichnet, daß der Teststreifen mit Hilfe der Teststreifenpackung gemäß Anspruch 7 so zur Sonne ausgerichtet wird, daß der Lichteinfall möglichst senkrecht zu der Matrix mit einer photoaktiven, chromogenen Substanz erfolgt.

14. Verfahren gemäß Anspruch 13, dadurch gekennzeichnet, daß der Teststreifen so auf eine oder beide parallelen Kanten, die eine reguläre Quaderkante des Behältnis der Teststreifenpackung vollständig oder teilweise ersetzen, gelegt wird, daß er in die Aufhängevorrichtung der Teststreifenpackung reicht.
